(19) Europäisches Patentamt
European Patent Office
Office européen des brevets

(11) Publication number: 0 330 874
A2

(12) # EUROPEAN PATENT APPLICATION

(21) Application number: 89102112.3

(22) Date of filing: 08.02.89

(51) Int. Cl.4: C12P 1/06 , A61K 35/66 , //(C12P1/06,C12R1:29)

The microorganisms have been deposited with the Agricultural Research Service Culture Collection (NRRL) under numbers 15839, 15975 and 18149.

(30) Priority: 29.02.88 US 161627
29.02.88 US 161625
29.02.88 US 161626

(43) Date of publication of application:
06.09.89 Bulletin 89/36

(84) Designated Contracting States:
AT BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: McGahren, William James
64 Glenwood Avenue
Demarest New Jersey 07627(US)
Inventor: Ellestad, George A.
59 Lt. Cox Drive
Pearl River New York 10965(US)

(74) Representative: Wächtershäuser, Günter, Dr.
Tal 29
D-8000 München 2(DE)

(54) **Antibacterial and antitumor agents LL-E33288epsilon-I and LL-E33288-epsilonBR, with processes and intermediates for producing said agents.**

(57) Antibacterial and antitumor agents designated LL-E33288ε-I and LL-E33288ε-Br, their production by strains of *Micromonospora echinospora* ssp. *calichensis* designated NRRL-15839, NRRL-15975 and NRRL-18149, and chemical processes for their production, are disclosed. Novel intermediates prepared in the chemical processes are disclosed. Processes for producing reductively aromatized derivatives of other antibiotics are also disclosed.

EP 0 330 874 A2

# ANTIBACTERIAL AND ANTITUMOR AGENTS LL-E33288$\epsilon$-I AND LL-E33288-$\epsilon$Br, WITH PROCESSES AND INTERMEDIATES FOR PRODUCING SAID AGENTS

## BACKGROUND OF THE INVENTION

The family of antibacterial and antitumor agents, known collectively as the LL-E33288 complex, are described and claimed in commonly-assigned European patent application, 85113751.3, filed October 29, 1985, which was published May 28, 1986, as EP 182,152.

This published application describes the LL-E33288 complex, the components thereof, namely LL-E33288$\alpha_1$-Br, LL-E33288$\alpha_1$-I, LL-E33288$\alpha_2$-Br, LL-E33288$\alpha_2$-I, LL-E33288$\alpha_3$-Br, LL-E33288$\alpha_3$-I, LL-E33288$\alpha_4$-Br, LL-E33288$\beta_1$-Br, LL-E33288$\beta_1$-I, LL-E33288$\beta_2$-Br, LL-E33288$\beta_2$-I, LL-E33288$\gamma_1$-Br, LL-E332887$\gamma_1$-I, and LL-E33288$\delta_1$-I, and methods for their production by aerobic fermentation utilizing a new strain of *Micromonospora echinospora* ssp *calichensis* or natural or derived mutants thereof.

Certain other antibiotics are pertinent to the instant invention, namely:

1) Esperamicin BBM-1675, a novel class of potent antitumor antibiotics. I. Physico-chemical data and partial structure. M. Konishi, et al., J. Antibiotics, 38, 1605 (1985). A new antitumor antibiotic complex. M. Konishi, et al., UK Patent application GB 2,141,425A, May 15, 1985.

2) New antitumor antibiotics, FR-900405 and FR-900406. I. Taxonomy of the producing strain. M. Iwami, et al., J. Antibiotics, 38, 835 (1985). New antitumor antibiotics FR-900405 and FR-900406. II. Production, isolation, characterization and antitumor activity. S. Kiyoto, et al., J. Antibiotics, 38, 840 (1985).

3) PD 114759 and PD 115028, novel antitumor antibiotics with phenomenal potency. I. Isolation and characterization. R. R. Bunge, et al., J. Antibiotics, 37, 1566 (1984). Biological and biochemical activities of the novel antitumor antibiotic PD 114759 and related derivatives. D. W. Fry, et al., Investigational New Drugs, 4, 3 (1986).

4) New antibiotic complex CL-1577A and CL-1577B produced by *Streptomyces* sp. ATCC 39363. European Patent application 0,132,082,A2.

5) CL-1577D and CL-1577E Antibiotic antitumor compounds, their production and use. U. S. Patent 4,539,203.

6) CL-1724 Antibiotic compounds, their production and use. U. S. Patent 4,554,162.

## SUMMARY OF THE INVENTION

This invention is concerned with a new component, LL-E33288$\epsilon$, derived by aerobic fermentation of the new strain of *Micromonospora echinospora* ssp *calichensis* or natural or derived mutants thereof. LL-E33288$\epsilon$ has antibacterial and antitumor activity.

Since, as is the case with other components of the LL-E33288 complex, iodine containing components are found only in fermentations using media containing inorganic or organic iodide, while bromine containing components are found only in fermentations using media containing inorganic or organic bromine, this inven tion encompasses both LL-E33288$\epsilon$-I, the iodine containing component and LL E33288$\epsilon$-Br, the bromine containing component. This invention is also concerned with two processes for the synthetic derivation of LL-E33288$\epsilon$-I and LL-E33288$\epsilon$-Br from the corresponding iodo or bromo LL-E332888$\gamma_1$ derivative.

## BRIEF DESCRIPTION OF THE DRAWINGS

FIGURE I is the proton magnetic resonance spectrum of LL-E33288$\epsilon$-I.

FIGURE II is the carbon-13 magnetic resonance spectrum of LL-E33288$\epsilon$-I.

FIGURE III is the proton magnetic resonance spectrum of LL-E33288-dethiomethyl$\gamma_1$-I.

## DESCRIPTION OF THE INVENTION

The physico-chemical characteristics of LL-E33288$\epsilon$-I are described below:

a) Molecular formula: $C_{54}H_{74}N_3O_{21}IS_2$;

b) Elemental analysis: C, 48.37; H, 5.72; N, 3.04; S, 4.98 and I, 9.28;

c) Optical rotation: $[\alpha]_D^{25}$ = -14±1° (C, 1.115%, methanol);

d) Optical density: 0.23 at 10µg/ml in methanol at 230nm;

e) Proton magnetic resonance spectrum: as shown in Figure I (300MHz, $CDCl_3$), with significant peaks at 7.16 (doublet), 7.21 and 7.26 (triplets) and 7.55ppm (doublet); and

f) Carbon-13 magnetic resonance spectrum: as shown in Figure II (75.46MHz, $CDCl_3$), with significant peaks in the regions of 120-160 and 190-210 ppm.

While the structures of LL-E33288$\epsilon$-I and Br have not been fully elucidated, proposed structures are given below.

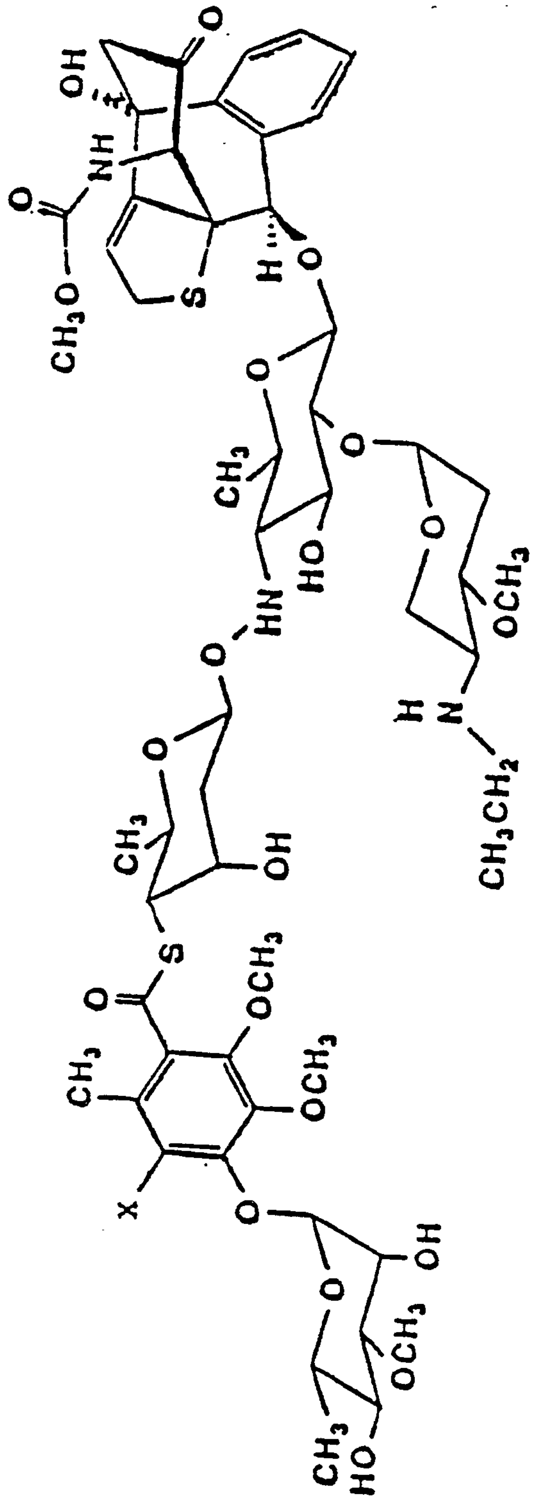

LL-E33288ε-I (X = I)
LL-E33288ε-Br (X = Br)

The new antibacterial and antitumor agents LL-E33288ε-I and LL-E33288ε-Br are formed during the cultivation, under controlled conditions of *Micromonospora echinospora* ssp *calichensis* NRRL 15839, 15975 and 18149.

These new microorganisms are maintained in the culture collection of the Medical Research Division, American Cyanamid Company, Pearl River, NY as culture numbers LL-E33288(NRRL 15839), LL-E33288-R66 (NRRL 15975) and LL-E33288 UV 784 (NRRL 18149). Viable cultures of these new microorganisms have been deposited with the Culture Collection Laboratory, Northern Regional Research Center, U. S. Department of Agriculture, Peoria, IL and have been added to its permanent collection.

Culture LL-E33288 (NRRL 15839) was isolated from a caliche clay soil sample collected in Texas.

Cultures LL-E33288-R66(NRRL 15975) and LL-E33288 UV 784 (NRRL 18149) were obtained as hereinafter described.

The generic assignment of NRRL 15839 to the genus *Micromonospora* was confirmed morphologically and chemically. The strain produces monospores either singly or in masses on the vegetative hyphae. No aerial hyphae were observed. Electron microscopic examination showed that the spores were warty. Whole cell analysis showed that the strain contained the *meso* isomer of diaminopimelic acid. The 3-OH derivative of diaminopimelic acid was present in large (major) amounts. Additionally the strain showed the presence of xylose plus traces of arabinose in its whole cell sugar hydrolysates (whole cell sugar pattern of Type D).

From macromorphological and physiological studies it was concluded that NRRL 15839 can be considered a subspecies of *M. echinospora* (it is closest to *M. echinospora* ssp. *pallida*). Data on the morphology of NRRL 15839 are given in Tables I and II. Physiological data are given in Tables III and IV.

TABLE I

| Macromorphology of NRRL 15839 (Colors are NBS-ISCC) | | | |
|---|---|---|---|
| ISP Agar Medium | Spores | Vegetative Mycelium | Soluble Pigments |
| Yeast-Malt (ISP 2) | - | Dark orange-yellow (72) | - |
| Oatmeal (ISP 3) | - | Colorless to pale orange-yellow (73) | - |
| Inorganic Salts-Starch (ISP 4) | Slight border of black spores | Dark orange-yellow (72) to light yellow-brown (76) | Light brownish |
| Glycerol-Asparagine (ISP 5) | - | Pale orange-yellow (73) to colorless | - |

TABLE II

| Macromorphology of NRRL 15839 on Various Agar Media Used for Actinomycete Growth (28°, 2 weeks) | |
|---|---|
| Agar Medium | NRRL 15839 |
| Pablum | Beige vegetative hyphae; Slight black spores; No soluble pigment |
| Yeast Czapek's | Beige vegetative hyphae; No spores; No soluble pigment |
| Czapek's | Beige vegetative hyphae; Slight black spores; No soluble pigment |
| Yeast Dextrose | Tan vegetative hyphae; Moderate black spores; Slight dark pigment |
| Nutrient | Colorless to tan vegetative hyphae; Slight black spores; No soluble pigment |
| Nutrient Glycerol | Colorless to light beige vegetative hyphae; No spores; No soluble pigment |
| Bennett's Dextrin | Colorless to beige vegetative hyphae; Slight black spores; Slight rose-brown pigment |
| Glucose Asparagine | Colorless to light orange-beige vegetative hyphae; No spores; No soluble pigment |

TABLE III

| Carbohydrate Utilization of NRRL 15839 | |
|---|---|
| Arabinose | + |
| Cellulose | - |
| Fructose | + |
| Glucose | + |
| Inositol | - |
| Mannitol | - |
| Raffinose | ± |
| Rhamnose | + |
| Sucrose | + |
| Xylose | + |

TABLE IV

Physiological Reactions of NRRL 15839

| | |
|---|---|
| **Hydrolysis of** | |
| Casein | + |
| Xanthine | - |
| Hypoxanthine | - |
| Tyrosine | + |
| Adenine | - |
| Gelatin | + |
| Potato Starch | + |
| Esculin | + |
| **Production of** | |
| Nitrate Reductase | + |
| Phosphatase | weak |
| Urease | - |
| **Growth on** | |
| Salicin | - |
| 5% Sodium Chloride | - |
| Lysozyme Broth | - |
| **Decarboxylation of** | |
| Acetate | + |
| Benzoate | - |
| Citrate | - |
| Lactate | - |
| Malate | - |
| Mucate | - |
| Oxalate | - |
| Propionate | + |
| Pyruvate | + |
| Succinate | - |
| Tartrate | - |

TABLE IV (continued)

| | |
|---|---|
| **Acid from** | |
| Adonitol | - |
| Arabinose | + |
| Cellobiose | + |
| Dextrin | + |
| Dulcitol | - |
| Erythritol | - |
| Fructose | + |
| Galactose | variable |
| Glucose | + |
| Glycerol | - |
| Inositol | - |
| Lactose | - |
| Maltose | + |
| Mannitol | - |
| Mannose | + |
| α-Methyl-D-glucoside | - |
| Melibiose | - |
| Raffinose | + |
| Rhamnose | + |
| Salicin | + |
| Sorbitol | - |
| Sucrose | + |
| Trehalose | + |
| Xylose | + |
| β-Methyl-D-xyloside | - |
| **Growth at** | |
| 10°C | - |
| 42°C | + |
| 45°C | + |

+ = positive; - = negative

Two antibiotic-producing mutants were derived from the original culture LL-E33288 (NRRL 15839) in accordance with the following description and diagram. The original culture LL-E33288 (NRRL 15839) was plated and 50 single colonies were isolated. These were designated NS1 to NS50 (NS = natural selection).

Fermentation of these isolates showed that those with moderate sporulation were generally better producers of the LL-E33288 complex. Isolate NS6 was selected as representative of this group.

Using isolate NS6 as the starting culture, spore suspensions were prepared and exposed to various mutagens. From a series of exposures to ultraviolet irradiation, single colonies were obtained, from which isolate UV610 was selected as a high yielding mutant. Isolate UV610 was streaked and subisolates 1 to 7 were obtained. Subisolate UV610(3) was selected for further work.

Vegetative growth from isolate UV610(3) (see following diagram) was prepared as employed for fermentation and used to inoculate a flask of medium consisting of peptone, dextrose, molasses and water. This medium was supplemented with LL-E33288$\beta_1$-Br at a concentration of 8$\mu$g/ml. A number of platings were done from this flask and a resistant population was obtained on the seventh day. A total of 97 colonies (R1 to R97) were isolated. Isolate R66 was subsequently deposited as NRRL 15975.

Isolate R80, which is essentially similar to R66 in its biosynthetic potential, was used as the starting culture from which a spore suspension was prepared and exposed to relatively high concentrations of the LL-E33288 complex, in order to obtain higher yielding isolates which were resistant to the LL-E33288 antibiotics. One survivor, labeled T2, did produce higher yields of LL-E33288$\beta_1$-Br and LL-E33288$\gamma_1$-I in flask fermentations.

A spore suspension of T2 was prepared and exposed to ultraviolet irradiation. A total of 131 colonies were then isolated (UV703 to UV834), fermented and assayed. Isolate UV784 was selected for its relatively high yield and was subsequently deposited as NRRL 18149.

## Derivation Diagram

```
                 E-33288 (Wild type)(NRRL 15839)
                  |Natural selection
                  v
                 NS6
                  |Ultraviolet irradiation
                  v
                 UV610
                  |Natural selection
                  v
                 UV610(3)
                  |Exposure to LL-E33288beta1-Br
       ------------------------------
       |                            |
       v                            v
      R80                          R66 (NRRL 15975)
       |Exposure to
       |LL-E33288 complex
       v
      T2
       |Ultraviolet irradiation
       v
     UV784 (NRRL 18149)
```

It is to be understood that for the production of LL-E33288$\epsilon$-I and LL-E33288$\epsilon$-Br, the present invention is not limited to the above described organisms or to organisms fully answering the above growth and microscopic characteristics which are given for illustrative purposes only. In fact, it is desired and intended to include the use of mutants produced from these organisms by various means such as exposure to x-radiation, ultraviolet radiation, N′-methyl-N′-nitro-N-nitrosoguanidine, actinophages and the like.

The in vitro antibacterial activity of LL-E33288$\epsilon$-I was determined against a spectrum of gram-positive and gram-negative bacteria by a standard agar dilution method. Mueller-Hinton agar containing two-fold decreasing concentrations of the antibiotic were poured into petri plates. The agar surfaces were inoculated with 1 to 5x10$^4$ colony forming units of bacteria by means of a Steer replicating device. The lowest

concentration of LL-E33288ε-I that inhibited growth of a bacterial strain after about 18 hours growth at 35°C was recorded as the minimal inhibitory concentration (MIC) for that strain. The results appear in Table V.

TABLE V

In vitro Antibacterial Activity of LL-E33288ε-I

| Organism | | Minimal Inhibitory Concentration (μg/ml) |
|---|---|---|
| *Escherichia coli* | CMC 84-11 | >64 |
| *Escherichia coli* | No. 311-(MP) | >64 |
| *Escherichia coli* | ATCC 25922 | >64 |
| *Klebsiella pneumoniae* | CMC 84-5 | >64 |
| *Klebsiella pneumoniae* | AD (MP) | >64 |
| *Enterobacter cloacae* | CMC 84-4 | >64 |
| *Enterobacter aerogenes* | IO 83-44 | >64 |
| *Serratia marcescens* | CMC 83-27 | >64 |
| *Serratia marcescens* | F 35 (MP) | >64 |
| *Morganella morganii* | IO 83-18 | >64 |
| *Providencia stuartii* | CMC 83-82 | >64 |
| *Citrobacter diversus* | K 82-24 | >64 |
| *Citrobacter freundii* | IO 83-13 | >64 |
| *Acinetobacter* sp | CMC 83-89 | >64 |
| *Acinetobacter* sp | IO 83-49 | >64 |
| *Pseudomonas aeruginosa* | 12-4-4 (MP) | >64 |
| *Pseudomonas aeruginosa* | ATCC 27853 | >64 |

TABLE V (continued)

| Organism | | Minimal Inhibitory Concentration(μg/ml) |
|---|---|---|
| *Staphylococcus aureus* | Smith | 4 |
| *Staphylococcus aureus* | SSC 82-21 | 4 |
| *Staphylococcus aureus* | ATCC 25923 | 4 |
| *Staphylococcus aureus* | SSC 82-20 | 4 |
| *Staphylococcus aureus* | SSC 82-23 | 4 |
| *Staphylococcus aureus* | SSC 82-24 | 4 |
| *Staphylococcus aureus* | SSC 82-54 | 4 |
| *Staphylococcus epidermidis* | CMC 83-133 | 4 |
| *Staphylococcus epidermidis* | ATCC 12228 | 4 |
| *Streptococcus faecalis* | ATCC 29212 | 4 |
| *Streptococcus faecalis* | VGH 84-65 | 4 |
| *Streptococcus faecalis* | CMC 83-53 | 4 |
| *Streptococcus faecalis* | UCI 85-20 | 4 |
| *Streptococcus faecalis* | IO 83-28 | 4 |

It has now been discovered that antibacterial and antitumor agents LL-E33288$\epsilon$-I and LL-E33288$\epsilon$-Br may also be synthetically derived by reductive aromatization of the corresponding iodo or bromo LL-E33288$\gamma_1$ derivative with a reagent such as triphenylphosphine, β-mercaptoethanol, reduced glutathione, 1,4-dithiothreitol or a variety of other sulfhydryl-containing reagents in a solvent such as dichloromethane, acetonitrile, ethyl acetate, methanol, ethanol or chloroform. The yield and product depend to a considerable degree on the polarity of the reagent and solvent. In addition, increasing the polarity of the reagent serves to increase the speed of the reaction. In a preferred embodiment, 1-4-dithiothreitol is the reagent and acetonitrile is the solvent.

It has further been determined that treatment of the aforementioned antibiotics LL-E33288$\alpha_1$-Br, LL-E33288$\alpha_1$-I, LL-33288$\alpha_2$-Br, LL-33288$\alpha_2$-I, LL-33288$\alpha_3$-Br, LL-E33288$\alpha_3$-I, LL-E33288$\alpha_4$-Br, LL-33288$\beta_1$-Br, LL-E33288$\beta_1$-I, LL-E33288$\beta_2$-Br, LL-E33288$\beta_2$-I and LL-E33288$\delta_1$-I, with the above reagents results in the production of the corresponding reductively aromatized antibiotic.

It has further been determined that treatment of the aforementioned antibiotics BBM-1675, FR-900405, FR-900406, PD 114759, PD 115028, CL-1577A, CL-1577B, CL-1577D, CLE-1577E and CL-1724, with the above reagents results in the production of the corresponding reductively aromatized antibiotic.

While the structures of LL-E33288 components have not been fully elucidated, proposed structures are given below to illustrate the reaction scheme.

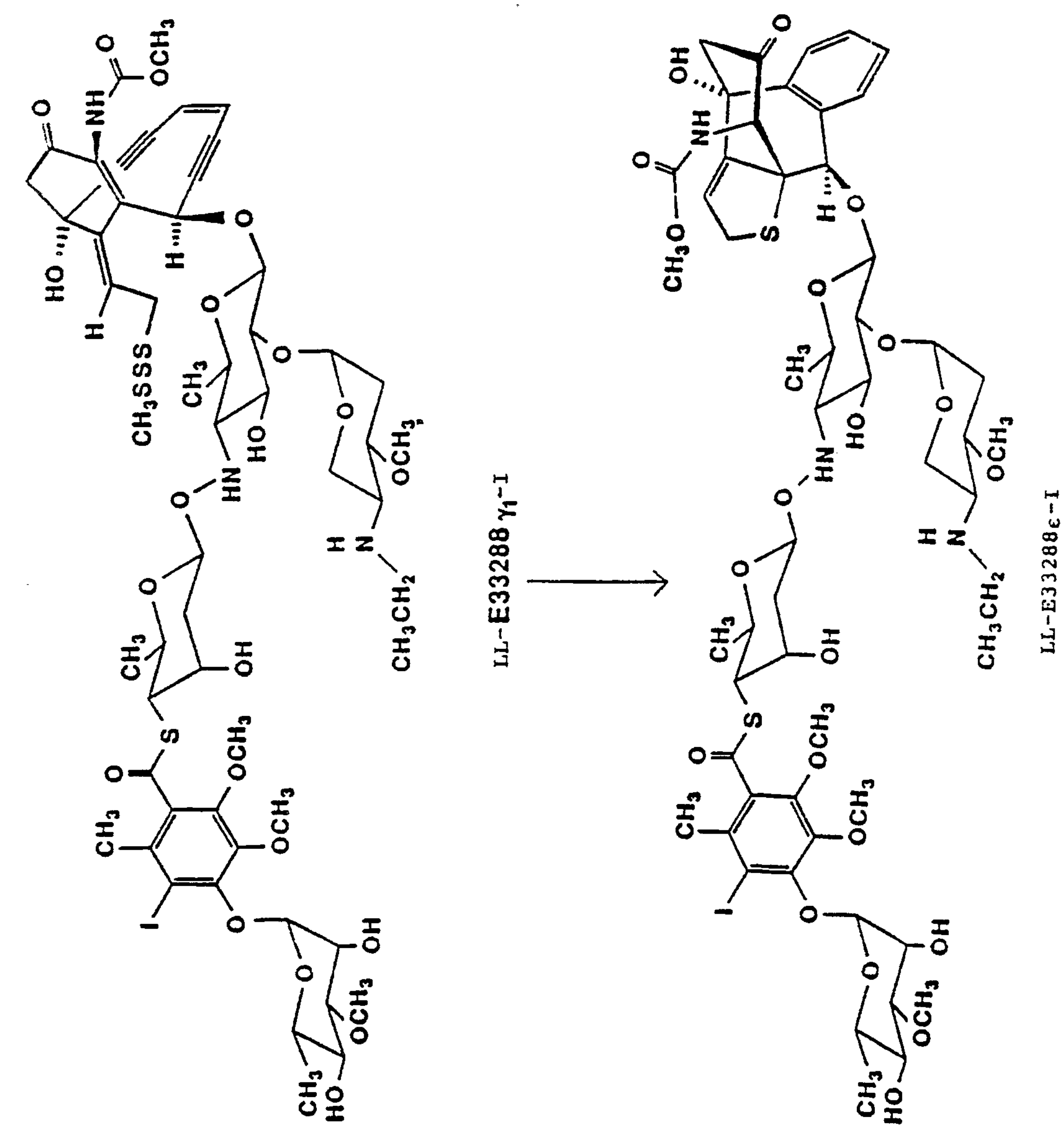

LL-E33288$\gamma_1$-I

LL-E33288$\epsilon$-I

This invention is further concerned with a synthetic process for producing LL-E33288$\epsilon$-I and LL-E33288$\epsilon$-Br from LLE33288$\gamma_1$-I and LL-E33288$\gamma_1$-Br and with intermediates developed during this process, called dethiomethyl$\gamma_1$ or dimers, which have antitumor activity of their own.

These reactions are represented schematically below and proposed structures for LL-E33288$\epsilon$-I and Br, LL-E33288$\gamma_1$-I and Br, LL-E33288$\delta_1$-I and the iodo and bromo dimers are given.

LL-E33288$\gamma_1$-I + TRIPHENYLPHOSPHINE $\xrightarrow[0^{o}C]{Dichloromethane}$

1

Dethiomethyl$\gamma_1$-I + TRIPHENYLPHOSPHINE $\xrightarrow[D]{Methanol}$

2

LL-E33288$\epsilon_1$-I

3

This invention is also concerned with the dethiomethyl derivative of LL-E33288$\delta_1$-I which may be prepared by the following reaction sequence.

LL-E33288$\delta_1$-I + TRIPHENYLPHOSPHINE $\xrightarrow[0^{o}C]{Dichloromethane}$

Dethiomethyl$\delta_1$-I

Additionally, the same reaction may be used to prepare dethiomethyl$\delta_1$-Br from LL-E33288$\delta_1$-Br.

In accordance with the above reaction scheme LL-E33288$\gamma_1$-I 1 is dissolved in dichloromethane cooled in an ice bath and treated with a solution of triphenylphosphine in dichloromethane giving dethiomethyl-$\gamma_1$-I 2., which is purified by reverse-phase, preparative high performance liquid chromatography using a column from Separations Technology, dissolved in methanol and treated with triphenylphosphine, with slight warming to produce LL-E33288$\epsilon$-I.

The reaction of LL-E33288$\gamma_1$-Br using the above sequence produces dethiomethyl-$\gamma_1$-Br and then LL-E33288$\epsilon$-Br. Similarly, the same sequence may be used to convert dethiomethyl$\delta_1$-I to LL-E33288$\epsilon_1$-I and to convert dethiomethyl$\delta_1$-Br to LL-E33288$\epsilon_1$Br.

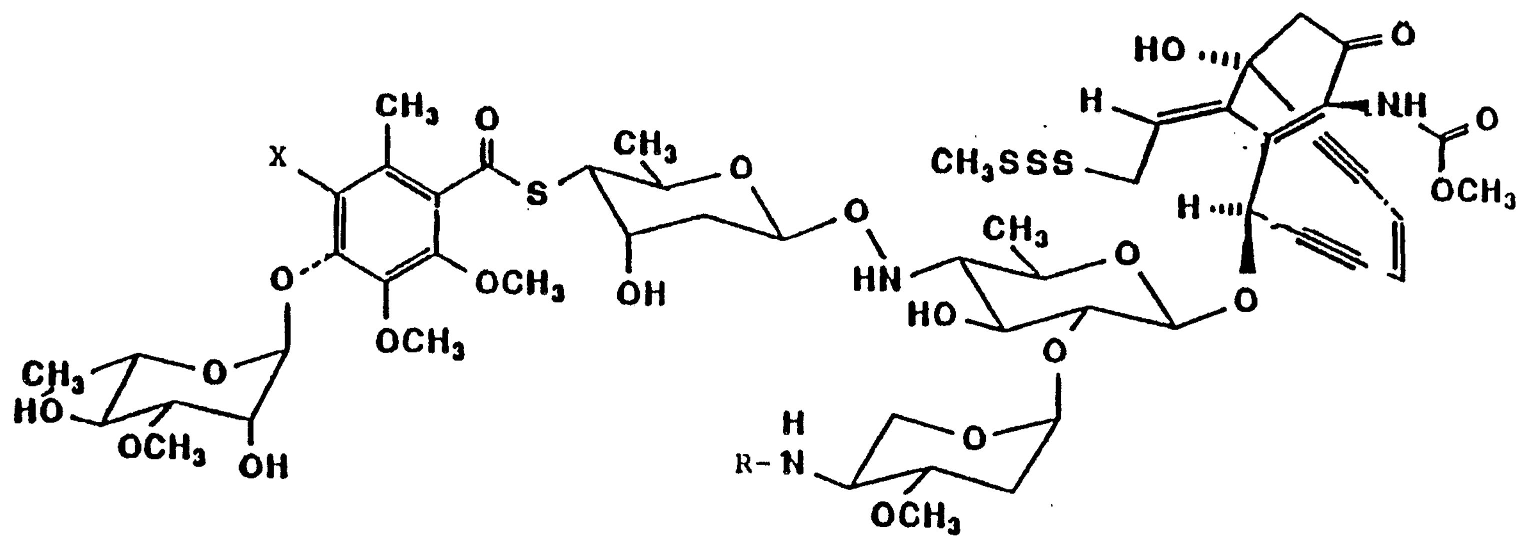

Proposed Structures for LL-E33288$\gamma_1$-I (X = I, $R=CH_2CH_3$)
LL-E33288$\gamma_1$-Br (X = Br, $R=CH_2CH_3$),
and LL-E33288$\delta_1$-I (X=I, $R=CH_3$)

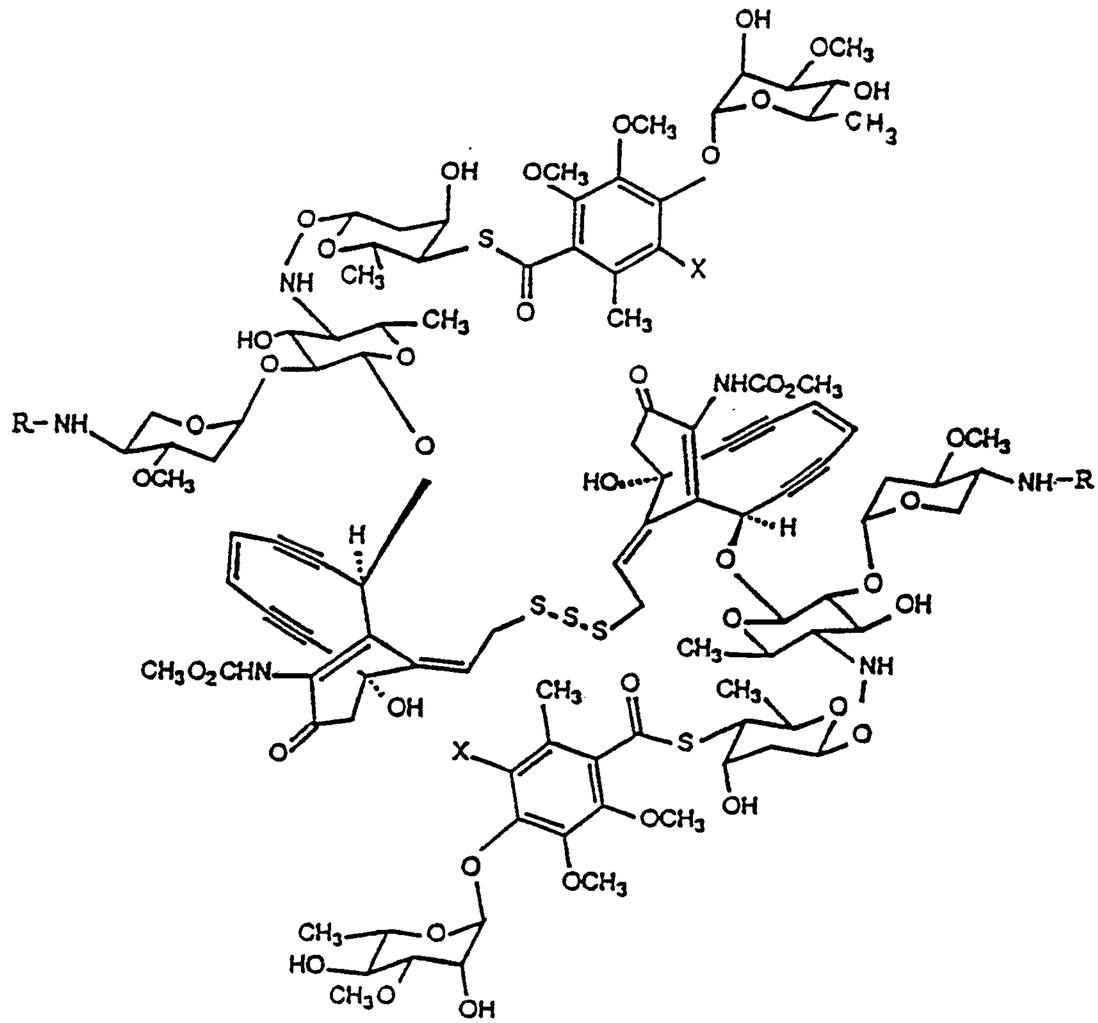

Dethiomethyl $\gamma_1$-I ($X = I$, $R=CH_2CH_3$)

Dethiomethyl $\gamma_1$-Br ($X = Br$, $R=CH_2CH_3$)

Dethiomethyl $\delta_1$-I ($X=I$, $R=CH_3$)

The physical-chemical characteristics of dethiomethyl$\gamma_1$-I are:

a) Elemental analysis: C, 46.46; H, 5.80; N, 3.01; I, 9.10; and S, 5.74;

b) Molecular formula: $C_{108}H_{142}N_6O_{42}I_2S_5$;

c) Molecular weight: 2608.4 (FABMS); and

d) a proton magnetic resonance spectrum as shown in Figure III (300MHz, $CDCl_3$).

The in vitro antibacterial activity of dethiomethyl$\gamma_1$-I was determined against a spectrum of gram-positive and gram-negative bacteria by a standard agar dilution method. Mueller-Hinton agar containing two-fold decreasing concentrations of the antibiotic were poured into petri plates. The agar surfaces were inoculated with 1 to $5x10^4$ colony forming units of bacteria by means of a Steers replicating device. The lowest concentration of dethiomethyl$\gamma_1$-I that inhibited growth of a bacterial strain after about 18 hours growth at 35°C was recorded as the minimal inhibitory concentration (MIC) for that strain. The results appear in Table VI.

TABLE VI

*In vitro* Antibacterial Activity of dethiomethyl$\gamma_1$-I

| Organism | | Minimal Inhibitory Concentration($\mu$g/ml) |
|---|---|---|
| *Escherichia coli* | CMC 84-11 | 4 |
| *Escherichia coli* | No. 311-(MP) | 4 |
| *Escherichia coli* | ATCC 25922 | 2 |
| *Klebsiella pneumoniae* | CMC 84-5 | 8 |
| *Klebsiella pneumoniae* | AD (MP) | 2 |

TABLE VI (continued)

| Organism | | Minimal Inhibitory Concentration(μg/ml) |
|---|---|---|
| *Enterobacter cloacae* | CMC 84-4 | 8 |
| *Enterobacter aerogenes* | IO 83-44 | 8 |
| *Serratia marcescens* | CMC 83-27 | 4 |
| *Serratia marcescens* | F 35 (MP) | 4 |
| *Morganella morganii* | IO 83-18 | 4 |
| *Providencia stuartii* | CMC 83-82 | 8 |
| *Citrobacter diversus* | K 82-24 | 4 |
| *Citrobacter freundii* | IO 83-13 | 2 |
| *Acinetobacter* sp | CMC 83-89 | 4 |
| *Acinetobacter* sp | IO 83-49 | 4 |
| *Pseudomonas aeruginosa* | 12-4-4 (MP) | 4 |
| *Pseudomonas aeruginosa* | ATCC 27853 | 4 |
| *Staphylococcus aureus* | Smith | $\leq 4 \times 10^{-6}$ |
| *Staphylococcus aureus* | SSC 82-21 | $3 \times 10^{-5}$ |
| *Staphylococcus aureus* | ATCC 25923 | $4 \times 10^{-3}$ |
| *Staphylococcus aureus* | SSC 82-20 | $2.5 \times 10^{-4}$ |
| *Staphylococcus aureus* | SSC 82-23 | $6 \times 10^{-5}$ |
| *Staphylococcus aureus* | SSC 82-24 | $\leq 4 \times 10^{-6}$ |
| *Staphylococcus aureus* | SSC 82-54 | $3 \times 10^{-5}$ |
| *Staphylococcus epidermidis* | CMC 83-133 | $1.2 \times 10^{-4}$ |
| *Staphylococcus epidermidis* | ATCC 12228 | $1 \times 10^{-3}$ |
| *Streptococcus faecalis* | ATCC 29212 | $1.5 \times 10^{-2}$ |
| *Streptococcus faecalis* | VGH 84-65 | $1.5 \times 10^{-2}$ |
| *Streptococcus faecalis* | CMC 83-53 | $2.5 \times 10^{-1}$ |
| *Streptococcus faecalis* | UCI 85-20 | $1.5 \times 10^{-2}$ |
| *Streptococcus faecalis* | IO 83-28 | $1.5 \times 10^{-2}$ |

Certain *in vivo* testing systems and protocols have been developed by the National Cancer Institute for testing compounds to determine their suitability as antineoplastic agents. These have been reported in "Cancer Chemotherapy Reports", Part III, Volume 3, No. 2 (1972), Geran, *et al*. These protocols have established standardized screening tests which are generally followed in the field of testing for antitumor agents. Of these systems, lymphocytic leukemia P388 is particularly significant to the present invention. This neoplasm is utilized for testing as transplantable tumors in mice. Significant antitumor activity shown in this protocol by a percentage increase of mean survival times of the treated(T) animals over the control(C) animals is indicative of similar results in human leukemias and solid tumors.

Lymphocytic Leukemia P388 Test

The animals used were BDFI mice, all of one sex, weighing a minimum of 17 g and all within a 3 g weight range. There were 5 or 6 mice per test group. The tumor transplant was by intraperitoneal injection of 0.5 ml of dilute ascitic fluid containing $10^6$ cells of lymphocytic leukemia P388. The test compounds were administered intraperitoneally at a volume of 0.5 ml in 0.2% Klucel in normal saline on days 1, 5 and 9 (relative to tumor inoculation) at the indicated doses. The mice were weighed and survivors recorded on a regular basis for 30 days. The median survival time and the ratio of survival time for treated(T)/control(C) animals were calculated. The positive control compound for LL-E33288$\epsilon$-I was 1,4-dihydroxy-5,8-bis[[2-(2-hydroxyethylamino)ethyl]amino]anthraquinone, dihydrochloride (U. S. Patent 4,197,249). The positive control compound for dethiomethyl-$\gamma_1$-I was 1,4-bis[2-(2-hydroxyethylamino)ethylamino]-5,8-dihydroxyanthroquinone dihydrochloride, Lederle Laboratories, Pearl River, New York ("Novantrone"). The controls were given as an intraperitoneal injections in 0.5 ml of 0.2% Klucel on days 1, 5 and 9 at the indicated doses. The results appear in Table VII.

If T C X 100 (%) is 125 or over, the tested compound is considered to have significant anti-tumor activity.

TABLE VII

| Lymphocytic Leukemia P388 Test | | | |
|---|---|---|---|
| Compound | Dose (mg/kg) | Median Survival (Days) | T/C x 100 (%) |
| LL-E33288$\epsilon$-I | 3.2 | 12 | 120 |
| | 1.6 | 16.5 | 165 |
| | 0.8 | 17.5 | 175 |
| | 0.4 | 15.5 | 155 |
| | 0.2 | 15.5 | 155 |
| | 0.1 | 15 | 150 |
| | 0.05 | 13 | 130 |
| Control | - | 10 | - |
| Positive Control | 1.6 | 27 | 270 |
| | 0.8 | >30 | >300 |
| | 0.4 | 21.5 | 215 |
| Dethiomethyl-$\gamma_1$-I | 0.025 | 23.5 | 235 |
| | 0.0125 | 22 | 220 |
| | 0.0062 | 19.5 | 195 |
| | 0.0031 | 18.5 | 185 |
| | 0.00155 | 16 | 160 |
| | 0.008 | 13 | 130 |
| | 0.0004 | 12.5 | 125 |
| Control | - | 10 | - |
| Positive Control | 1.6 | 27 | 270 |
| | 0.8 | >30 | >300 |
| | 0.4 | 21.5 | 215 |

The invention is further illustrated by the Examples set forth below which are not intended to limit the invention.

## Example 1

Inoculum Preparation

A typical medium used to grow the primary inoculum was prepared according to the following formula:

| | |
|---|---|
| Dextrin | 2.4% |
| Glucose | 0.5% |
| Yeast extract | 0.5% |
| Tryptone | 0.5% |
| Beef extract | 0.3% |
| Calcium carbonate | 0.4% |
| Antifoam agent | 0.3% |
| Water | balance to 100% |

This medium was sterilized and a 100 ml portion in a flask inoculated with mycelia of the culture NRRL 18149. This medium was placed on a rotary shaker and agitated vigorously for 48 hours at 32° C. This primary inoculum was then used to inoculate 10 liters of the above sterile medium in a bottle. This medium was agitated at 32° C for 48 hours providing secondary inoculum. This secondary inoculum was then used to inoculate 300 liters of the above sterile medium in a tank which was incubated at 32° C for 48 hours with agitation by an impeller driven at 220 rpm and a sterile air flow of 200 liters per minute, providing tertiary inoculum.

Example 2

Tank Fermentation

A fermentation medium was prepared according to the following formula:

| | |
|---|---|
| Sucrose | 2.0 % |
| Ferric sulfate heptahydrate | 0.01% |
| Magnesium sulfate heptahydrate | 0.02% |
| Peptone | 0.5 % |
| Molasses | 0.5 % |
| Potassium iodide* | 0.05% |
| Calcium carbonate | 0.5 % |
| Antifoam agent | 0.3 % |
| Water | balance to 100 % |

*Substitution of potassium bromide will result in the production of bromo derivatives.

A 2700 liter portion of the above medium was sterilized and then inoculated with 300 liters of tertiary inoculum prepared as described in Example 1. Aeration was supplied at the rate of 6.5 liters of sterile air per liter of mash per minute and agitation was supplied by an impeller driven at 120 rpm. The temperature was maintained at 30° C and the fermentation was terminated after about 125 hours, at which time the mash was harvested.

Example 3

Isolation of LL-E33288ε-I

A total of 2400 liters of mash from fermentations conducted as described in Example 2 was agitated for one hour with an equal volume of ethyl acetate. The resulting emulsion was filtered through diatomac eous earth in a press. The ethyl acetate phase was separated, evaporated to about 200 liters under reduced pressure and adjusted to pH 6 to 7 with 1N sodium hydroxide. The ethyl acetate phase was separated and concentrated to 30-40 liters, then diluted with 30 liters of water to ease the emulsion, agitated and then allowed to settle, forming three phases. The aqueous (bottom phase) was separated and extracted with ethyl acetate. This extract was combined with the clear (upper) solvent phase and concentrated to about 6 liters.

The 6 liters of oily suspension was divided into 1 liter portions, each of which was concentrated to an oil and defatted by partitioning between hexane and methanol. The methanol phases were evaporated to an oil stage and then combined. This oil was reconstituted in dichloromethane, charged on 2 kg of silica gel in a 12 inch bed column and developed using 4 liters of dichloromethane, followed by 4 liters each of 2.5% methanol in dichloromethane and 5% methanol in dichloromethane. One liter fractions were collected every 20 minutes. The activity was found in fractions 9 to 14 by biochemical induction assay. These fractions were combined and evaporated to 24 g of crude dark brown solid.

This solid was divided into 4 portions of about 6 g each. Each portion was stirred in 100 ml of acetonitrile for 30 minutes, then filtered. The filtrate was diluted with 150 ml of 0.2M ammonium acetate solution and refiltered. This filtrate was pumped onto a Waters LC 500 instrument containing a Prepak reverse phase cartridge, which had been equilibrated with acetonitrile:0.2m ammonium acetate solution (45:55). The column was developed using 7 to 8 liters of the same solvent. The elution process was monitored at 254nm. The second liter of eluate contained LL-E33288ε-I. This eluate was evaporated sufficiently to remove the bulk acetonitrile. The remaining aqueous suspension was extracted with one half its volume of ethyl acetate. The ethyl acetate extract was dried over anhydrous magnesium sulfate, concentrated to 10-20 ml and dripped into 75 ml of vigorously stirred hexane. The resulting precipitate was collected and dried, giving 1.694 g of LL-E33288ε-I.

Example 4

Purification of LL-E33288ε-I

A 1.69 g portion of the LL-E33288ε-I from Example 3 was rechromatographed on a Prepak reverse phase column using the solvent system acetonitrile:0.2m ammonium acetate solution (37:63). The core fractions of the symmetrical peak were taken and processed, giving 960 mg of off-white solid.

An 880 mg portion of the above solid was subjected to reverse phase preparative chromatography using the same developing solvent solution. The main peak of the monitored profile was shaved in two portions. The front fraction was processed, giving 186 mg of pure LL-E33288ε-I.

Example 5

Preparation of LL-E33288ε-I by Treatment of LL-E33288$\gamma_1$-I with Triphenylphosphine

A solution of 1.67 g of LL E33288$\gamma_1$-I in 100 ml of dichloromethane was treated with a solution of 500 mg of triphenylphosphine in 5 ml of dichloromethane. This mixture was stirred for 2 hours and then filtered. The filtrate was taken to dryness, the residue dissolved in methanol and treated with 268 mg of triphenylphosphine with warming to effect solution. The reaction was then stirred for 16 hours at room temperature and filtered. The filtrate was evaporated to dryness and then chromatographed on a 60 x 3 cm (LH-20; Pharmacia) column, eluting with hexane:dichloromethane:methanol (2:1:1). The fractions containing

LL-E33288$\epsilon$-I were combined providing 336 mg of partially purified product which was then rechromatographed on the same column. Fractions containing LL-E33288$\epsilon$-I were identified using thin layer chromatography ("TLC"). The TLC plates were placed in ethyl acetate saturated with a buffer of 0.2M dipotassium hydrogen phosphate containing 10% isopro panol. The plates were then ultraviolet quenched to identify the fractions of interest(fractions 11-15). Fractions 11-15 (5 ml each) were combined, concentrated to dryness and taken up in a small amount of ethyl acetate. This was added dropwise to an excess of stirred hexane and the precipitate collected, giving 112 mg. A 100 mg portion was then chromatographed on a preparative reverse phase high performance liquid chromatography column, eluting with acetonitrile:0.2M ammonium acetate buffer (37:63). The active fractions were identified based on their retention times on the column. The LL-E33288$\epsilon$ fractions were combined, the acetonitrile evaporated, the active component extracted into ethyl acetate and precipitated with hexane, giving 20 mg of pure LL-E33288$\epsilon$-I. The identity of the product was confirmed by comparison with the product obtained from natural sources as described above.

## Example 6

### Preparation of LL-E33288$\epsilon$-Br by Treatment of LL-E33288$\gamma$-Br with Triphenylphosphine

A portion of LL-E33288$\gamma_1$-Br is reacted following the procedure described in Example 5, giving LL-E33288$\epsilon$-Br.

## Example 7

### Preparation of LL-E33288$\epsilon$-I by Treatment of LL-E33288$\gamma_1$-I with Dithiothreitol

A solution of 100 mg of LL-E33288$\gamma_1$-I in 8 ml of acetonitrile was treated with 59 mg of 1,4-dithiothreitol. The mixture was stirred for 3 hours and then filtered. The filtrate was concentrated to dryness, taken up in dichloromethane containing a small amount of methanol and chromatographed on silica gel, eluting successively with 2, 4 and 5% methanol in dichloro methane. Fractions 7, 8 and 9 were combined and evaporated. The residue was precipitated from hexane/ethyl acetate, giving 30 mg of pure LL-E33288$\epsilon$-I.

## Example 8

### Preparation of LL-E33288$\epsilon$-Br by Treatment of LL-E33288$\gamma_1$-Br with Dithiothreitol

A portion of LL-E33288$\gamma_1$-Br is reacted following the procedure described in Example 7, giving LL-E33288$\epsilon$-Br.

## Example 9

### Preparation of Dethiomethyl$\gamma_1$-I

One gram of LL-E33288$\gamma_1$-I in 50 ml of dichloromethane was cooled in an ice bath and treated with 293 mg of triphenylphosphine in 2 ml of dichloromethane. After stirring for 2 hours at ice bath temperature, the solution was allowed to warm to room temperature and the particulate dethiomethyl$\gamma_1$-I collected. This product was purified by preparative HPLC using a 1 x 14 inch Separations Technology column packed with $C_{18}$ reverse phase support (25μ) with the solvent system acetonitrile:0.2M ammonium acetate (48:52). Fractions containing dethiomethyl$\gamma_1$-I were identified using thin layer chromatography ("TLC"). The TLC plates were placed in ethyl acetate saturated with a buffer of 0.2M dipotassium hydrogen phosphate containing 10% isopropanol. The plates were then ultraviolet quenched to identify the fractions of interest (fractions 5 and 6). Fractions 5 and 6 were combined, the acetonitrile removed in vacuo and the resultant cloudy solution extracted with ethyl acetate. The ethyl acetate solution was washed with water, dried, concentrated to a small volume and added dropwise to 100 ml of hexane with stirring. The pre cipitate was collected and dried, giving 168 mg of dethiomethyl$\gamma_1$-I.

## Example 10

### Conversion of Dethiomethyl$\gamma_1$-I to LL-E33288$\epsilon$-I

To a solution of 42 mg of dethiomethyl$\gamma_1$-I in 10 ml of methanol was added 10 mg of triphenylphosphine. The mixture was warmed and then stirred for 16 hours at room temperature. A 12 mg portion of triphenylphosphine was added followed by warming at 40-50 °C for one hour. The resulting LL-E33288$\epsilon$-I was purified by preparative TLC using Whatman 1000μ silica gel plates. Extraction of the LL-E33288$\epsilon$-I zone with ethyl acetate gave 4 mg of pure product.

## Claims

1. The compound LL-E33288$\epsilon$-I, having:
   a) a molecular formula: $C_{54}H_{74}N_3O_{21}IS_2$;
   b) an elemental analysis: C, 48.37; H, 5.72; N, 30.4; S, 4.98 and I, 9.28;
   c) an optical rotation: $[\alpha]_D^{25}$ = -14±1° (C, 1.115%, methanol);
   d) an optical density: 0.23 at 10μg/ml in methanol at 230nm;
   e) a proton magnetic resonance spectrum as shown in Figure I of the drawings; and
   f) a carbon-13 magnetic resonance spectrum: as shown in Figure II of the drawings.

2. A process for producing LL-E33288$\epsilon$-I as recited in Claim 1, which comprises
   a) aerobically fermenting the organism *Micromonospora echinospora* ssp *calichensis* NRRL 15839 or its antibiotic producing mutants NRRL 15975 or NRRL 18149 in a liquid medium containing assimilable sources of carbon, nitrogen, iodine and inorganic salts, until substantial antibiotic activity is imparted to said medium and then recovering the antibiotic therefrom;
   b) reacting LL-E33288$\gamma_1$-I with triphenylphosphine or a sulfhydryl-containing reagent in a solvent, followed by chromatographic separation and purification of the LL-E33288$\epsilon$-I;
   c) reacting LL-E33288$\gamma_1$-I with triphenylphosphine in dichloromethane at ice bath temperature, followed by warming to room temperature, collecting the so formed dethiomethyl$\gamma_1$-I, reacting the dethiomethyl$\gamma_1$-I with triphenylphosphine in methanol with slight warming and collection of the so formed LL-E33288$\epsilon$-I; or
   d) reacting LL-E33288$\delta_1$-I with triphenylphosphine in dichloromethane at ice bath temperature, followed by warming to room temperature, collecting the so formed dethiomethyl$\delta_1$-I, reacting the dethio methyl$\delta_1$-I with triphenylphosphine in methanol with slight warming and collection of the so formed LL-E33288$\epsilon$-I.

3. A process according to Claim 2, wherein the organism is *Micromonospora echinospora* ssp *calichensis* NRRL 18149 and wherein the sulfhydryl-containing reagent is selected from the group consisting of β-mercapto-ethanol, reduced glutathione or 1,4-dithiothreitol andthe solvent is selected from the group consisting ofdichloramethane, acetonitrile, ethyl acetate, methanol,ethanol or chloroform.

4. A process for producing LL-E33288$\epsilon$-Br, which comprises

a) aerobically fermenting the organism *Micromonospora echinospora* ssp *calichensis* NRRL 15839 or its antibiotic-producing mutants NRRL 15975 or NRRL 18149 in a liquid medium containing assimilable sources of carbon, nitrogen, bromine and inorganic salts, until substantial antibiotic activity is imparted to said medium and then recovering the antibiotic therefrom;

b) reacting LL-E33288$\gamma_1$-Br with triphenylphosphine or a sulfhydryl-containing reagent in a solvent, followed by chromatographic separation and purification of the LL-E33288$\epsilon$-Br;

c) reacting LL-E33288$\gamma_1$-Br with triphenylphosphine in dichloromethane at ice bath temperature, followed by warming to room temperature, collecting the so formed dethiomethyl$\gamma_1$-Br, reacting the dethiomethyl$\gamma_1$-Br with triphenyl phosphine in methanol with slight warming and collection of the so formed LL-E33288$\epsilon$-Br; or

d) reacting LL-E33288$\delta_1$-Br with triphenyl phosphine in dichloromethane at ice bath temperature, followed by warming to room temperature, collecting the so formed dethiomethyl$\delta_1$-Br, reacting the dethiomethyl$\delta_1$-Br with triphenyl phosphine in methanol with slight warming and collection of the so formed LL-E33288$\epsilon$-Br.

5. A process according to Claim 4, wherein the organism is *Micromonospora echinospora* ssp *calichensis* NRRL 18149 and wherein the sulfhydryl-containing reagentis selected from the group consisting of $\beta$-mercapto-ethanol, reduced glutathione or 1,4-dithiothreitol andthe solvent is selected from the group consisting ofdichloromethane, acetonitrile, ethyl acetate, methanol,ethanol or chloroform.

6. The compound LL-E33288$\epsilon$-Br when prepared by the process of Claim 4.

7. The compound dethiomethyl$\gamma_1$-I which has:

a) a molecular formula: $C_{108}H_{142}N_6O_{42}I_2S_5$;

b) an elemental analysis: C, 46.46; H, 5.80; N, 3.01; I, 9.10; and S, 5.74;

c) a molecular weight: 2608.4 (FABMS); and

d) a proton megnetic resonance spectrum as shown in Figure III.

8. The compound dethiomethyl$\gamma_1$-I when produced by the reaction of LL-332881$\gamma_1$-I with triphenylphosphine in dichloromethane at ice bath temperature.

9. The compound dethiomethyl$\gamma_1$-Br when produced by the reaction of LL-E33288$\gamma$-Br with triphenylphosphine in dichloromethane at ice bath temperature.

10. The compound dethiomethyl$\delta_1$-I when produced by the reaction of LL-332881$\delta_1$-I with triphenylphosphine in dichloromethane at ice bath temperature.

11. The compound dethiomethyl$\delta_1$-Br when produced by the reaction of LL-E33288$\delta$-Br with triphenylphosphine in dichloromethane at ice bath temperature.

12. A process for producing a reductively aromatized derivative of a compound selected from LL-E32288$\alpha_1$-Br, LL-E33288$\alpha_1$-I, LL-E33288$\alpha_2$-Br, LL-E33288$\alpha_2$-I, LL-E33288$\alpha_3$-Br, LL-E33288$\alpha_3$-I, LL-E33288$\alpha_4$-Br, LL-E33288-$\beta_1$-Br, LL-E33288$\beta_1$-I, LL-33288$\beta_2$-Br, LL-E33288$\beta_2$-I, LL-E33288$\delta_1$-I, BBM-1675, FR-900405, FR-900406, PD 114759, PD 115028, CL- 1577A, CL-1577B, CL-1577D, CL-1577E and CL-1724 which comprises reacting one of said antibiotics with triphenylphosphine or a sulfhydryl-containing reagent in a solvent followed by chromatographic separation and purification.

13. The process of claim 12 wherein the sulfhydryl-containing reagent is selected from the group consisting of $\beta$-mercaptoethanol, reduced glutathione or 1,4-dithiothreitol and the solvent is selected from the group consisting of dichloromethane, acetonitrile, ethyl acetate, methanol, ethanol or chloroform.

14. A method of treating bacterial infections in a warm-blooded animal which comprises administering to said animal an antibacterially effective amount of a compound selected from LL-E33288$\epsilon$-I as recited in Claim 1, or LL-E33288$\epsilon$-Br as recited in Claim 6.

15. A method of inhibiting the growth of tumors in a warm-blooded animal which comprises administering to said animal a tumor inhibiting amount of a compound selected from LL-E33288$\epsilon$-I as recited in Claim 1, or LL-E33288$\epsilon$-Br as recited in Claim 6.

16. A method of regressing leukemia in a warm-blooded animal which comprises administering to said animal an antileukemic amount of a compound selected from LL-E33288$\epsilon$-I as recited in Claim 1, or LL-E33288$\epsilon$-Br as recited in Claim 6.

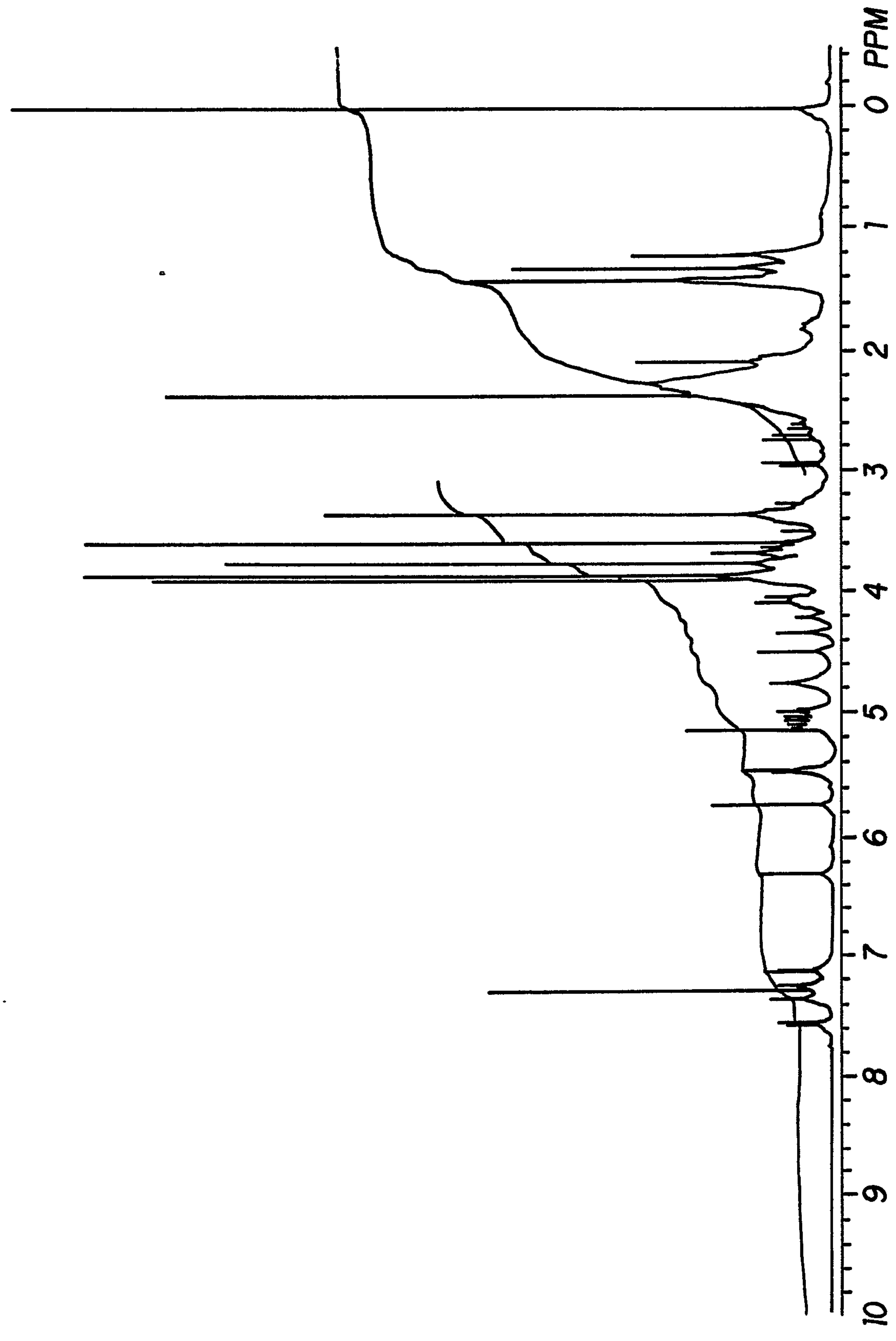


FIG. 1

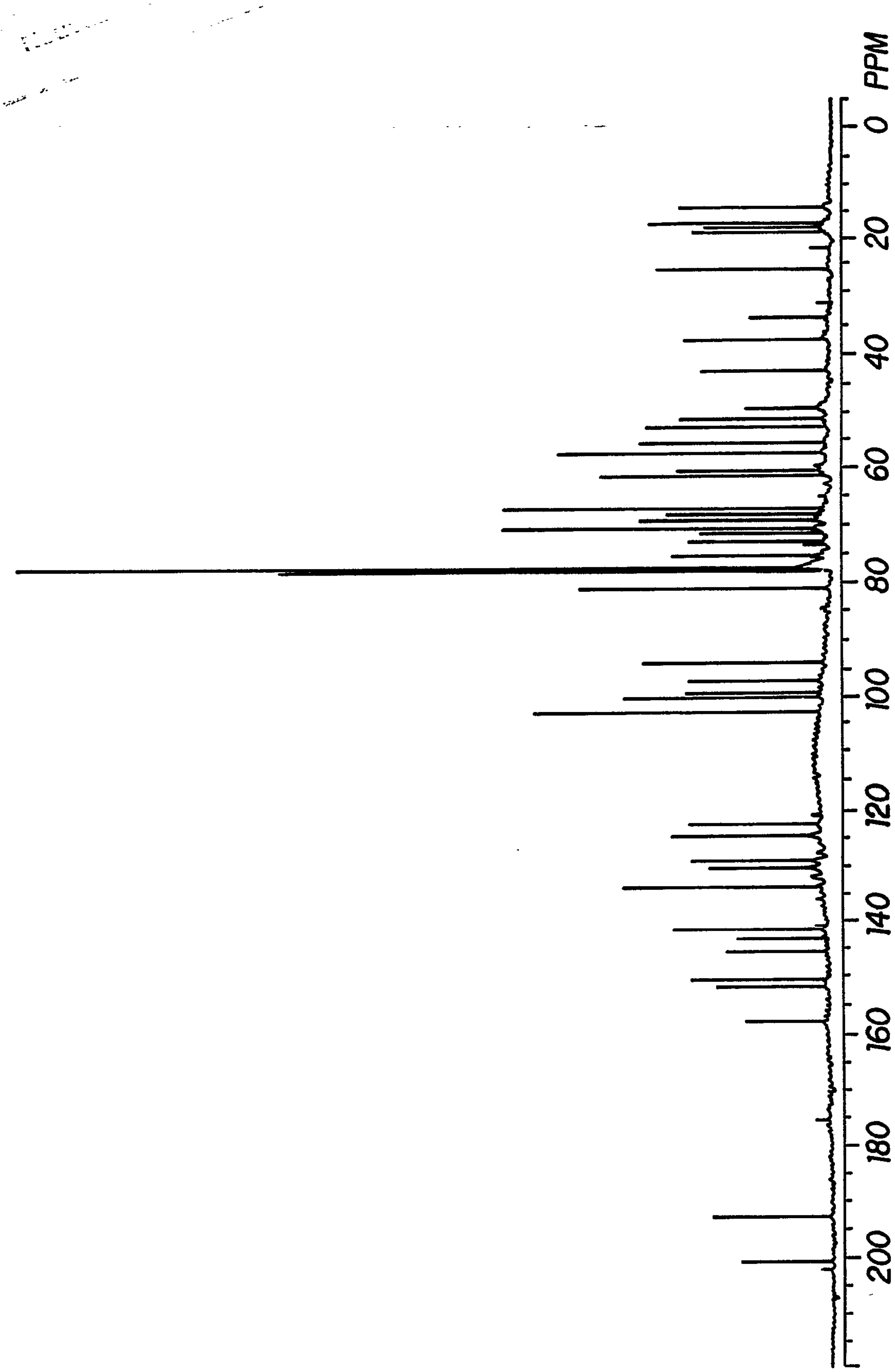


FIG. 2

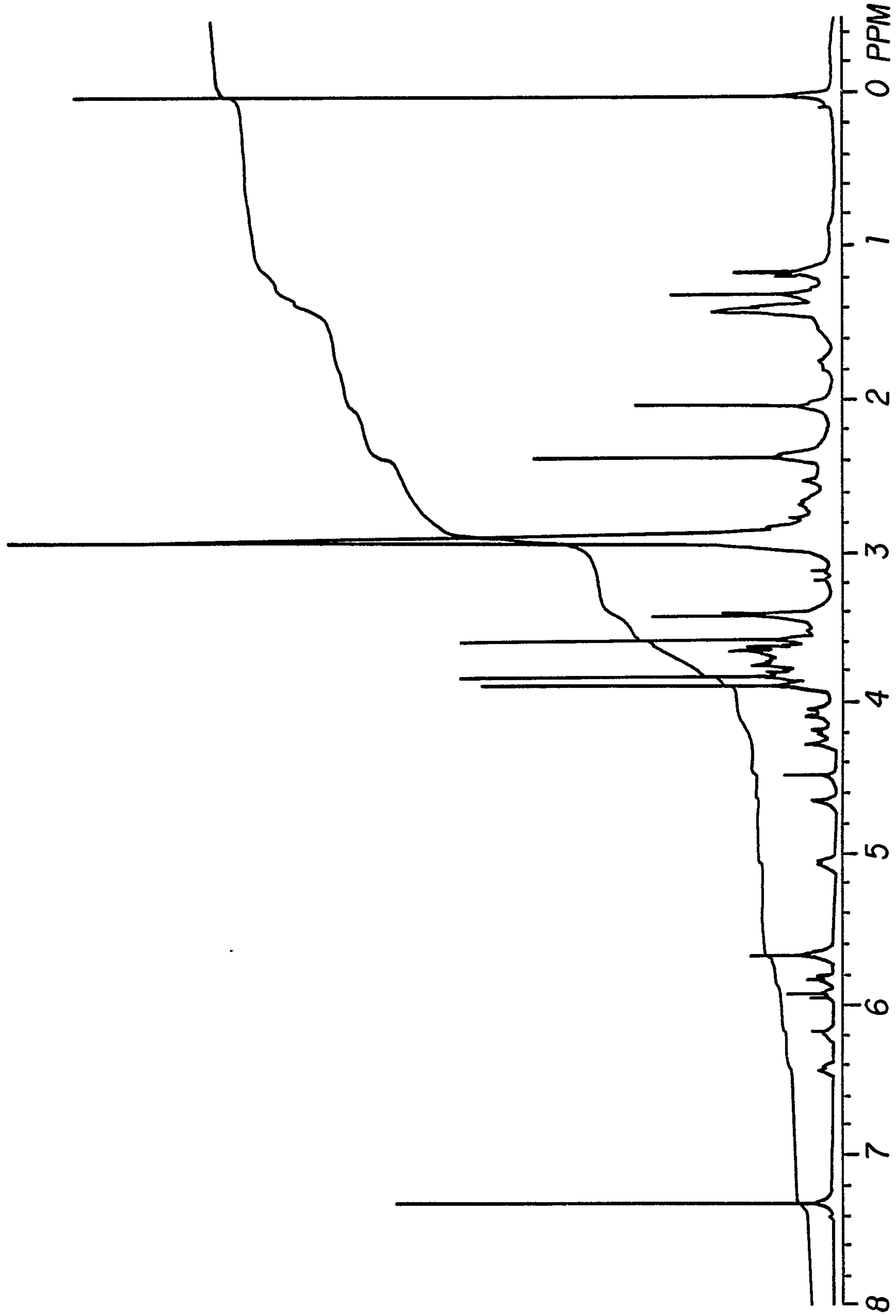


FIG. 3